# EUROPEAN PATENT SPECIFICATION

(11) **EP 4 082 534 B1**
(45) Date of publication and mention of the grant of the patent: **18.11.2026**
(21) Application number: 20906098.7
(22) Date of filing: 24.12.2020
(51) Int. Cl.: A61K 9/20, A61K 9/22, A61K 9/28, A61K 9/30, A61K 9/50, A61K 31/519, A61P 19/02, A61P 29/00, A61P 1/00, A61P 1/04, A61P 11/06, A61P 17/00, A61P 17/14, A61P 25/28, A61P 3/10, A61P 35/00, A61P 35/02, A61P 37/02

(54) **SOLID PREPARATION, AND PREPARATION METHOD THEREFOR AND USE THEREOF**
PHARMAZEUTISCHE ZUSAMMENSETZUNG UND HERSTELLUNGSVERFAHREN DAFÜR SOWIE VERWENDUNG DAVON
PRÉPARATION SOLIDE, SON PROCÉDÉ DE PRÉPARATION ET SON UTILISATION

(30) Priority: 25.12.2019 CN 201911355327
(43) Date of publication of application: 02.11.2022
(73) Proprietor: Sinotherapeutics Inc., Shanghai 201210 (CN)
(72) Inventor: QIAN, Xiaoming, Shanghai 201210 (CN); FANG, Larry Yun, Shanghai 201210 (CN); WAN, Jiansheng, Shanghai 201210 (CN); LI, Zhao, Shanghai 201210 (CN); LI, Kun, Shanghai 201210 (CN)
(74) Representative: Reitstötter Kinzebach
(86) International application number: PCT/CN2020/138988
(87) International publication number: WO 2021/129735

(56) References cited:
- WO-A1-2005/027878
- WO-A1-2012/100949
- WO-A1-2014/147526
- WO-A2-2015/184087
- CN-A- 103 520 164
- CN-A- 103 585 126
- CN-A- 103 585 126
- CN-A- 106 074 357
- CN-A- 106 074 357
- CN-A- 106 074 357
- CN-A- 106 138 012
- CN-A- 108 201 534
- US-A1- 2006 057 197
- YOUNIS USIR S ET AL: "Preformulation and Evaluation of Tofacitinib as a Therapeutic Treatment for Asthma", AAPS PHARMSCITECH, SPRINGER INTERNATIONAL PUBLISHING, CHAM, vol. 20, no. 5, 16 April 2019 (2019-04-16), pages 1 - 11, XP036761356, DOI: 10.1208/S12249-019-1377-0
- LAMBA M ET AL: "Model-Informed Development and Registration of a Once-Daily Regimen of Extended-Release Tofacitinib", vol. 101, no. 6, 9 February 2017 (2017-02-09), US, pages 745 - 753, XP093096101, ISSN: 0009-9236, Retrieved from the Internet <URL:https://api.wiley.com/onlinelibrary/tdm/v1/articles/10.1002%2Fcpt.576> DOI: 10.1002/cpt.576

## Description

### Technical Field

The present disclosure relates to a solid formulation and a preparation process and use thereof.

### Background

In the pharmaceutical field, development of a suitable dosage form for active pharmaceutical ingredient (API) is always the focus of the pharmaceutical field. For the dosage form of a drug, the dissolution of a pharmaceutical ingredient can reflect the rate and degree of drug's release in the body, and to a certain extent, the therapeutic effect on the disease.

Taking Tofacitinib as an example, CN103845302A discloses a film-coated tablet of Tofacitinib with good properties. The film-coated tablet contains a film coating base agents, Tofacitinib and a pharmaceutical additive.

For another example, CN104622827A discloses a Tofacitinib tablet and a preparation process thereof. For the tablet, direct compression lactose and microcrystalline cellulose with good fluidity and compression formability are used as diluent agent and dry binder, and direct powder compression method with simple process is adopted for preparation.

CN105878202A discloses a Tofacitinib citrate tablet, which consists of Tofacitinib citrate and a pharmaceutically acceptable excipient, the excipient includes a filler, a disintegrant, a glidant and a coating agent, prepared by direct powder compression method.

CN105101952A discloses an oral sustained release formulation of Tofacitinib and a pharmaceutically acceptable salt thereof, which is a once daily pharmaceutical dosage form, comprising Tofacitinib or a pharmaceutically acceptable salt thereof and a pharmaceutically acceptable carrier, wherein the dosage form is a sustained release dosage form, and the dry granulation compression method is used in Example 1, Example 2 and the like to prepare the tablet core of the sustained release tablet.

CN 106074357 A relates to an improved preparation of ticagrelor or a pharmaceutical salt thereof for or once-daily administration. The plasma concentration can reach about 0.2 µg/mL or more within 2 hours; the plasma concentration can still reach about 0.2 µg/mL or more 12 hours later after taking the drug; and the maximum plasma concentration (Cmax) of ticagrelor or its pharmaceutical salt ranges from about 0.2 µg/mL to about 0.8 µg/mL. The preparation can reduce the number of administration times, thereby improving the compliance of patients with administration, reducing the risks of myocardial infarction or stroke caused by acute thrombosis due to patients missing taking ticagrelor.

CN 103585126 A relates to a tofacitinib composition and a preparation method thereof. Tofacitinib, as an active component, is combined with pharmaceutically acceptable excipients and prepared into the tofacitinib composition by adopting the existing preparation technique. The preparation technique accords with the production requirements and can guarantee the stability and safety of the preparation. The tofacitinib composition is convenient for transportation and preservation, and provides a novel medicine selection for adults in the active phase and patients with moderate to severe rheumatoid arthritis not responding well to methotrexate therapy.

Younis Usir S et al, "Preformulation and Evaluation of Tofacitinib as a Therapeutic Treatment for Asthma" relates to preformulation studies on factors that affect the solubility, stability, and solid-state characteristics of tofacitinib citrate and its free base. The results facilitated the development of a nebulized formulation for inhalational delivery in BALB/c mice challenged with house dust mite allergens. Tofacitinib has a basic pKa of 5.2, and its stability depends on pH, ionic strength, and temperature. To maximize stability, minimize ionic strength and temperature, with an optimal pH range of 2.0-5.0, tofacitinib citrate can be nebulized at a suitable droplet size (1.2 ± 0.2 µm MMAD) and reduced BAL eosinophils and total protein concentrations in HDM challenged animals, indicating its potential as an effective eosinophilic asthma therapy.

Lamba M. et al: "Model-Informed Development and Registration of a Once-Daily Regimen of Extended-Release Tofacitinib" relates to the fact that Extended-release (XR) formulations reduce dosing frequency compared to conventional (e.g., immediate release (IR) formulations. Regulatory registration requires PK and clinical efficacy data. Using a tofacitinib case study, they illustrate a model-informed E-R approach to translate trial data without a phase III trial. E-R analyses used validated clinical endpoints from Phase II dose-response and non-clinical dose fractionation studies of the IR formulation. Average concentration was established as the PK parameter for tofacitinib efficacy. Along with demonstrated equivalence in total systemic exposure, these evaluations supported the FDA approval of tofacitinib XR once daily.

WO 2015184087 A2 provides methods of treatment for hematological malignancies involving synergistic combination of a JAK2 inhibitor and a glucocorticoid or a JAK2 inhibitor, thalidomide or thalidomide derivative, and a glucocorticoid. The compositions and methods provide an unexpected efficacy in the treatment for hematological disorders. The hematological disorders treated by the current invention include multiple myeloma, and may also include hematological disorders that are refractory to prior cancer treatments, or a relapsed hematologic disorder.

WO 2014147526 A1 relates to oral sustained release formulations of tofacitinib and pharmaceutical acceptable salts thereof. The formulations described herein have desirable pharmacokinetic characteristics.

CN 103520164 relates to a sustained-release preparation composed of ticagrelor, a pharmaceutically acceptable sustained-release material, and other pharmaceutically acceptable excipients. The preparation has an immediate-release part and a sustained-release part. It can be dual-layer tablets obtained by compression by using a double-part tableting machine, or tablets with sustained-release core and immediate-release coating, or sustained-release capsules composed of the immediate-release part and the sustained-release part. This provides rapid onset and prolonged effective medication concentration, achieving an ideal treatment effect.

CN 106138012 relates to a method for preparing isosorbide mononitrate sustained-release capsules. It involves preparing quick-release and sustained-release micropellets, mixing them, and encapsulating to obtain the product. This product exhibits stability, resolves crystallization issues, and avoids problems like caking, heated softening, solvent removal difficulties, and micropellet adhesion. Experimental investigations show the capsules are bioequivalent to original triturates.

CN 108201534 discloses a rucaparib oral sustained/controlled release pharmaceutical composition, comprising rucaparib in a form for improved dissolution and a matrix polymer for regulating the release rate. The in vivo absorption behavior, plasma drug concentration and PARP enzyme inhibitory level of the pharmaceutical composition can be controlled, thereby improving the drug load and/or oral absorption and/or bioavailability and/or plasma drug concentration control and/or enzyme inhibitory level control of the rucaparib, and the composition can be used alone or with other therapies for treating cancers.

US 2006057197 A1 relates to pharmaceutical dosage forms comprising: an absorption window active agent; a controlled release component comprising enteric-coated controlled release beads, wherein the enteric-coated release beads comprise at least two pH-sensitive polymer layers. The controlled-release dosage forms provide good bioavailability of absorption window active agents.

WO 2012100949 A1 relates to oral dosage forms comprising a JAK3 inhibitor, preferably tasocitinib, suitable for modified release, and processes of preparing such oral dosage forms.

WO 2005027878 A1 relates to a delayed release pharmaceutical formulation comprising a core containing an active agent (e.g., a drug) and a delayed release compression coating comprising a natural or synthetic gum applied onto the surface of the core.

### Summary

In one aspect, provided is a solid formulation, wherein the solid formulation comprises an immediate release portion and a sustained release portion, and comprises Tofacitinib or a pharmaceutically acceptable salt thereof as active ingredients, wherein the sustained release portion comprises a sustained release matrix material, wherein the sustained release matrix material is about 40%-80% of the weight of the sustained release portion; the immediate release portion comprises a film-forming material, wherein the film-forming material is about 40%-80% of the weight of the immediate release portion; the weight ratio of the active ingredient in the sustained release portion to the active ingredient in the immediate release portion is about 7:3-20:1.

In an embodiment, in the solid formulation according to the present disclosure, the weight ratio of the active ingredient in the sustained release portion to the active ingredient in the immediate release portion is about 7:3-19:1, preferably about 4:1-19:1, more preferably about 4:1-12:1, for example about 4:1-11:1, about 4:1-9:1 or about 4:1-10:1.

In a preferable embodiment, the active ingredient is Tofacitinib citrate.

In a preferable embodiment, the immediate release portion and the sustained release portion each independently comprise one or more pharmaceutical excipients selected from the group consisting of a sustained release matrix material, a film-forming material, a binder, a filler, a disintegrant, a lubricant.

In a specific embodiment, the solid formulation is in the form of a tablet, a granule, a pellet or a capsule. The tablet is preferably a bilayer tablet, a coated tablet or an osmotic pump tablet .

In another aspect, provided is a pharmaceutical composition, comprising the solid formulation according to the present disclosure. The pharmaceutical composition may comprise one or more pharmaceutically acceptable excipients.

In yet another aspect, provided is the solid formulation or the pharmaceutical composition according to the present disclosure for use in the treatment or prevention of a disease selected from lupus, multiple sclerosis, rheumatoid arthritis, psoriatic arthritis, type I diabetes and diabetic complications, cancer, asthma, atopic dermatitis, autoimmune thyroid disease, moderate-to-severe active ulcerative colitis, Crohns' disease, ankylosing spondylitis, juvenile idiopathic arthritis, immune system disorder baldness, vitiligo, systemic lupus erythematosus, Alzheimer's disease and leukemia.

### Brief Description of the Drawings

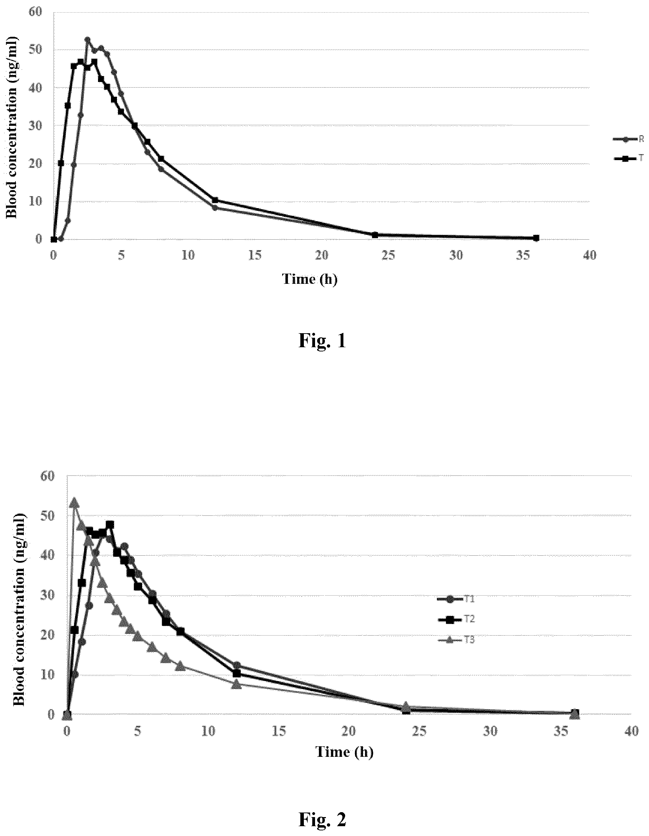

Figure 1 shows the Tofacitinib blood concentration-time curve after the single administration, wherein ● represents the commercial Tofacitinib sustained release tablet, and ■ represents the Tofacitinib coated tablet according to the present disclosure.
Figure 2 shows the Tofacitinib blood concentration-time curve after the single administration, wherein ● represents the Tofacitinib coated tablet 1-2 according to the present disclosure, ■ represents the Tofacitinib coated tablet 1-3 according to the present disclosure, ▲ represents the Tofacitinib coated tablet 1-6 according to the present disclosure.

### Detailed Description

The present disclosure will be described in detail below, and the description is provided for the purpose of illustration.

### General Definition and Terms

Unless otherwise stated, the technical and scientific terms used herein have the same meaning as commonly understood by a person skilled in the art. If there is a contradiction, the definition provided in this application shall prevail. When expressing a certain amount, concentration or other value or parameter in the form of a range, a preferable range, or a preferable range upper limit and a preferable range lower limit, it should be understood that it corresponds to specifically revealing any range by combining any pair of upper limit of the range or preferable range value with the lower limit of any range or preferable range value, regardless of whether the range is specifically disclosed. Unless otherwise stated, the numerical ranges listed herein are intended to include the endpoints of the range and all integers and fractions (decimals) within the range.

When used with a numerical variable, the term "approximate" or "about" usually refers to the value of the variable and all the values of the variable within the experimental error (for example, within an average 95% confidence interval) or within ± 10% of the specified value, or a wider range.

The term "metering ratio" refers to formulating various substances according to a certain weight. For example, the active ingredient and the sustained release material and plasticizer are formulated herein according to the specified weight ratio.

The term "optional" or "optionally" means the event described subsequent thereto may or may not happen. This term encompasses the cases that the event may or may not happen.

The expression "comprise" or its synonyms "contain", "include", "have" or the like are meant to be inclusive, which does not exclude other unlisted elements, steps or ingredients. The expression "consist of" excludes any unlisted elements, steps or ingredients. The expression "substantially consist of" refers to specified elements, steps or ingredients within a given range, together with optional elements, steps or components which do not substantively affect the basic and novel feature of the claimed subject matter. It should be understood that the expression "comprise" encompasses the expressions "substantially consist of" and "consist of".

The term "at least one" or "one or more" as used herein means one, two, three, four, five, six, seven, eight, nine or more.

The term "pharmaceutical acceptable" means application within the scope of sound medical judgment, suitable for use in contact with the tissues of subjects without undue toxicity, irritation, allergic response and the like, having reasonable benefit/risk ratio and effective for intended purpose.

The terms "active pharmaceutical ingredient", "active ingredient", "therapeutic agent", "active substance" or "active agent" refer to a chemical entity which is effective in treating or preventing a target disease or condition. In an embodiment, the active ingredient used in the formulation is Tofacitinib or a pharmaceutically acceptable salt thereof, particularly Tofacitinib citrate. Different portions of the formulations according to the present disclosure may use different forms of the active ingredient. In an embodiment of the present disclosure, the same active ingredient is used. In the present disclosure, a given compound and its corresponding other forms (such as salt, polymorph, solvate, cocrystal, amorphous form, anhydrous form or the like) belong to the same active ingredient, but can be considered as different forms or types.

Those skilled in the art will understand that when different forms or types of an active ingredient exist in a formulation, they need to be converted into their respective free form if the ratio is calculated. Taking Tofacitinib as an example, when Tofacitinib is contained in the immediate release portion and Tofacitinib citrate is contained in the sustained release portion, it is necessary to convert Tofacitinib citrate into Tofacitinib, or to convert Tofacitinib into Tofacitinib citrate. For another example, when the immediate release portion or the sustained release portion contains Tofacitinib and Tofacitinib citrate, it is necessary to convert Tofacitinib citrate into Tofacitinib, or to convert Tofacitinib into Tofacitinib citrate. For another example, when different types of salt forms of an active ingredient coexist in a solid formulation, it is necessary to convert the respective salt forms into corresponding free forms. In addition to the pharmaceutically acceptable salt, an active ingredient such as Tofacitinib, as used herein, also encompasses other forms of the active ingredient, such as polymorph, solvate (including, for example, a hydrate and a mixed solvate and a hydrate of a salt), cocrystal, amorphous form and anhydrous form and a mixture thereof. In a preferrable embodiment, the above forms are pharmaceutically acceptable.

The term "dissolution rate" refers to the dissolution rate of a drug from a solid formulation in a specified solvent.

The term "dissolution" refers to the rate and extent of dissolution of a drug from a solid formulation in a specified solvent.

The term "peak time of plasma drug concentration (Tₘₐₓ)" refers to the mean time when peak plasma drug concentration (Cₘₐₓ) is attained after drug administration. The term "peak plasma drug concentration (Cₘₐₓ)" refers to the mean peak plasma drug concentration attained after drug administration. The term "peak time of plasma drug concentration (Tₘₐₓ)" refers to the time when maximum plasma drug concentration (peak plasma drug concentration) is attained after drug administration. The term "AUC₀₋ₜ" refers to the mean integral area under a plasma drug concentration - time curve from the time point of 0 to t after drug administration.

In the present disclosure, the term "Ta value" refers to the time that the blood concentration can be maintained above 17 ng/mL (IC₅₀).

In the present disclosure, the term "strength" refers to the medicine strength, that is, the weight of the active pharmaceutical ingredient contained in each formulation unit, regardless of the form of the active ingredient, and the weight is that of the free form after conversion. Taking Tofacitinib as an example, in a Tofacitinib formulation with strength of 11 mg, when the active ingredient is Tofacitinib, the weight of Tofacitinib is 11 mg. For another example, in a Tofacitinib formulation with strength of 11 mg, when the active ingredient is Tofacitinib citrate, the weight of free Tofacitinib after conversion is 11 mg. For another example, in a Tofacitinib formulation with strength of 11 mg, when the active ingredient is Tofacitinib hydrochloride, the weight of free Tofacitinib after conversion is 11 mg.

### Solid formulation according to the present disclosure

In one aspect, provided is a solid formulation, comprising an immediate release portion and a sustained release portion, and comprises Tofacitinib or a pharmaceutically acceptable salt thereof as active ingredients, wherein the sustained release portion comprises a sustained release matrix material, wherein the sustained release matrix material is about 40%-80% of the weight of the sustained release portion; the immediate release portion comprises a film-forming material, wherein the film-forming material is about 40%-80% of the weight of the immediate release portion; the weight ratio of the active ingredient in the sustained release portion to the active ingredient in the immediate release portion is about 7:3-20: 1.

In the solid formulation of the present disclosure, the active ingredient is present in the immediate release portion and the sustained release portion of the formulation of the present disclosure, in the same or different forms (e.g., Tofacitinib or a pharmaceutically acceptable salt thereof or the like). For example, the active ingredient can be present in the form of a pharmaceutically acceptable salt in the immediate release portion and the sustained release portion, respectively. For another example, the active ingredient can be present in the immediate release portion in the form of a pharmaceutically acceptable salt and in the sustained release portion in the form of a free base. For another example, citrate salt of tofacitinib as an active ingredient can be present in the immediate release portion and the sustained release portion, respectively. For another example, citrate as an active ingredient can be present in the immediate release portion, and hydrochloride salt of tofacitinib as an active ingredient can be present in the sustained release portion,

As an example of the active ingredient used in the formulation of the present disclosure, Tofacitinib citrate, can be used. Therefore, in an embodiment of the present formulation, Tofacitinib citrate can serve as the active pharmaceutical ingredient (API).

Tofacitinib (chemical name of (3R,4R)-4-methyl-3-(methyl-7H-pyrrolo[2,3-d] pyrimidin-4-ylamino)-β-oxo-1-piperidinepropionitrile) is a JAK kinase inhibitor, by inhibiting the JAK pathway, reduces cytokine signaling, cytokine-induced gene expression and cell activation, thereby reducing various chronic inflammatory responses (see the following structure).

As a pharmaceutically acceptable salt of the used active ingredient, it can include, but are not limited to, a salt formed with an inorganic acid, a salt formed with an organic acid, or the like. Non-limiting examples of the salts formed with inorganic acid include but not limited to those formed with hydrochloric acid, hydrobromic acid, nitric acid, sulfuric acid, phosphoric acid or the like. Non-limiting examples of the salts formed with organic acid include but not limited to those formed with formic acid, acetic acid, trifluoroacetic acid, fumaric acid, oxalic acid, malic acid, maleic acid, tartaric acid, citric acid, succinic acid, methanesulfonic acid, benzenesulfonic acid, p-toluenesulfonic acid or the like. In a specifically preferable embodiment, the salt is citrate. In an embodiment of the salt, the active ingredient can be Tofacitinib citrate. Its structure is exemplified as follows:

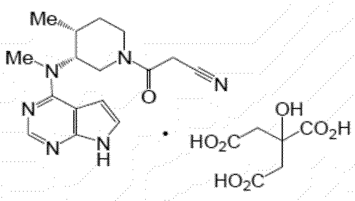

In the formulation of the present disclosure, the active ingredients are present in the immediate release portion and the sustained release portion. Alternatively, the active ingredients are present only in the immediate release portion and the sustained release portion. In the latter case, the calculation of the total amount of active ingredients in the formulation can be based on the sum of the active ingredients in the immediate release portion and the sustained release portion (i.e., the sum of the active ingredients in the sustained release portion and the immediate release portion can be 100%).

In the formulation of the present disclosure, the calculation of the active ingredients in the immediate release portion and the sustained release portion can be based on the weight of the active ingredients. Alternatively, as described above, the calculation of the active ingredients should be done according to conversion to a consistent form, and thus such a ratio can also correspond to a molar ratio of the active ingredients.

In an embodiment, the form of the active ingredient in the immediate release portion is identical to that in the sustained release portion, e.g., Tofacitinib citrate.

In a preferable embodiment, the weight ratio of the active ingredient in the sustained release portion to the active ingredient in the immediate release portion is about 7:3-19:1, preferably about 4:1-19:1, more preferably about 4:1-12:1. For example, the weight ratio is about 7:3, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, about 19:1, about 20:1, particularly about 4:1, about 7:3, about 9:1, and a range defined by any two of these ratios (values).

In another embodiment, the weight ratio of the active ingredient (e.g., Tofacitinib or a pharmaceutically acceptable salt thereof) in the sustained release portion to the active ingredient (e.g., Tofacitinib or a pharmaceutically acceptable salt thereof) in the immediate release portion can be about 7:3-19:1, for example about 19:1, about 47:3, about 93:7, about 23:2, about 91:9, about 9:1, about 89:11, about 22:3, about 87:13, about 43:7, about 17:3, about 21:4, about 83:17, about 41:9, about 81:19, about 4:1, about 79:21, about 39:11, about 77:23, about 19:6, about 3:1, about 37:13, about 73:27, about 18:7, about 71:29, about 7:3, and a range defined by any two of these ratios (values).

In an embodiment, the weight ratio of the active ingredient (e.g., Tofacitinib or a pharmaceutically acceptable salt thereof) in the sustained release portion to the active ingredient (e.g., Tofacitinib or a pharmaceutically acceptable salt thereof) in the immediate release portion is about 7:3-9: 1, for example about 9:1, about 89:11, about 22:3, about 87:13, about 43:7, about 17:3, about 21:4, about 83:17, about 41:9, about 81:19, about 4:1, about 79:21, about 39:11, about 77:23, about 19:6, about 3:1, about 37:13, about 73:27, about 18:7, about 71:29 or about 7:3, and a range defined by any two of these ratios (values).

In an embodiment, the weight ratio of the active ingredient (e.g., Tofacitinib or a pharmaceutically acceptable salt thereof) in the sustained release portion to the active ingredient (e.g., Tofacitinib or a pharmaceutically acceptable salt thereof) in the immediate release portion is about 39:11-41:9, for example about 41:9, about 81:19, about 4:1, about 79:21, about 39:11, and a range defined by any two of these ratios (values).

In a specific embodiment, the weight ratio of the active ingredient (e.g., Tofacitinib or a pharmaceutically acceptable salt thereof) in the sustained release portion to the active ingredient (e.g., Tofacitinib or a pharmaceutically acceptable salt thereof) in the immediate release portion is about 39:11-41:9, for example about 4:1.

### Form and composition of the formulation

The formulation of the present disclosure is in the form of a granule, a pellet, a tablet or a capsule.

In an embodiment of the formulation of the present disclosure, the sustained release portion and the immediate release portion can each independently be in the form of a granule, a pellet, a layer, a tablet (such as an osmotic pump tablet), a coating. As an example, the immediate release portion can also be in the form of a granule or a particulate comprised in the sustained release portion such as a sustained coating.

The formulation of the present disclosure can contain one or more sustained release portions and one or more immediate release portions. These sustained release portions and immediate release portions as well as the active ingredients and other ingredients therein can be independently selected.

Those skilled in the art will understand that the sustained release portion and immediate release portion as used herein should be understood broadly and not referring to only one specific component in the formulation. For example, a formulation such as a coated tablet or an osmotic pump tablet can contain a sustained tablet core and a coating thereon. When the coating is a sustained coating, it can also be considered a sustained release portion.

In an embodiment, the sustained release portion in the formulation is a sustained release granule. In another embodiment, the immediate release portion in the formulation is an immediate release granule.

In a preferrable embodiment, the immediate release granule and the sustained release granule can be filled in a capsule. In another preferrable embodiment, the immediate release granule and the sustained release granule can be filled in a pouch.

In an embodiment, the immediate release granule and the sustained release granule can be mixed and compressed into a tablet.

In an embodiment, the sustained release portion in the formulation is a coating sustained release granule or a matrix sustained release granule. In another embodiment, the immediate release portion in the formulation is located within or outside a coating film or outside a matrix sustained release granule.

In an embodiment, the sustained release portion in the formulation is a sustained release pellet. In another embodiment, the immediate release portion in the formulation is an immediate release pellet.

In a preferable embodiment, the immediate release pellet and the sustained release pellet can be filled in a capsule.

In a preferred embodiment, the immediate release pellet and the sustained release pellet can be filled in a pouch. In an embodiment, the immediate release pellet and the sustained release pellet can be mixed and compressed into a tablet.

In a specific embodiment, the formulation of the present disclosure may comprise a sustained release pellet, wherein the sustained release pellet is a coated sustained release pellet or a matrix sustained release pellet, and the immediate release portion is located inside or outside the coating film or outside the matrix sustained release pellet.

In an embodiment, the formulation of the present disclosure can be a bilayer tablet, wherein one layer is a sustained release layer (corresponding to the sustained release portion), and the other layer is an immediate release layer (corresponding to the immediate release portion). In a preferable embodiment, the sustained release layer is a matrix sustained release tablet. In another preferable embodiment, the sustained release layer is a membrane-controlled sustained release tablet. In a specifically preferable embodiment, the sustained release layer is an osmotic pump tablet.

In an embodiment, the formulation of the present disclosure can be a coated tablet, wherein the sustained release portion is a coated tablet core or a matrix tablet core, and the immediate release portion is located within or outside the coating film or outside the matrix tablet core. In a preferable embodiment, the coated tablet is an osmotic pump tablet.

In an embodiment, the formulation of the present disclosure can be a capsule, the content of which is a sustained release granule or pellet, and the immediate release portion is coated around the capsule or located within the capsule shell.

In a preferrable embodiment of the present formulation, the formulation is a coated tablet, wherein the sustained release portion is a coated tablet core, and the immediate release portion is a coating film, wherein the coated tablet core and coating film each independently contain the active ingredient of the present disclosure. Preferably, the coated tablet is an osmotic pump tablet.

In a preferable embodiment of the present formulation, the active ingredient is Tofacitinib citrate.

In an embodiment of the present formulation, the sustained release matrix material in the sustained release portion is selected from the group consisting of: hydroxypropyl methyl cellulose, hydroxypropyl cellulose, hydroxyethyl cellulose, polyoxyethylene, glyceryl behenate, polymethylmethacrylate, acrylic resin, povidone, xanthan gum, sodium alginate, calcium alginate, carbomer, chitin, polyethylene, polysiloxane.

The followings are examples of some available sustained release matrix materials, without limitation:
hydroxypropyl methyl cellulose, which can be selected from for example the commercial product Metolose SR 90SH of Shin-Etsu;
hydroxypropyl cellulose, which can be selected from for example the commercial products Klucel^{™} GF, Klucel^{™} JF, Klucel^{™} MF, Klucel^{™} HF of Ashland;
hydroxyethyl cellulose, which can be selected from for example commercial products Natrosol G, Natrosol M, Natrosol HX, Natrosol HHX of Ashland;
glyceryl behenate, which can be selected from for example the commercial product Compritol^{®} ATO888 of GATTEFOSSE.

In an embodiment, the sustained release matrix material in the sustained release portion is about 40%-50% or about 50%-70%, for example about 40%, about 45%, about 50%, about 55%, about 60%, about 65%, about 70% of the weight of the sustained release portion.

In an embodiment, the sustained release portion comprises a film-forming material. The film-forming material in the sustained release portion or in the immediate release portion can be one or more selected from the group consisting of: cellulose acetate, ethyl cellulose, hydroxypropyl methyl cellulose, acrylic resins.

The following are examples of some available film-forming materials, without limitation:
ethyl cellulose, which can be selected from for example the commercial products Aqualon^{™} ethyl cellulose of Ashland, ETHOCEL^{™} of Dupont;
hydroxypropyl methyl cellulose, which can be selected from for example the commercial product Metolose 60 SH of Shin-Etsu;
cellulose acetate, which can be selected from for example the commercial products CA-320S, CA-394-60LF, CA-398-3, CA-398-6, CA-398-10 and CA-398-30 of Eastman;
ethyl cellulose, which can be selected from for example the ETHOCEL^{™} Standard 10 Premium of Dupont.

In an embodiment, the sustained release portion comprises a film-forming material (membrane). The active ingredient may or may not be located in the film-forming material. The active ingredient may be an immediate release active ingredient or may be a sustained release active ingredient.

When the sustained release portion comprises a film-forming material, the film-forming material can be about 30%-70%, more preferably about 40%-67%, for example about 30%, about 35%, about 40%, about 45%, about 50%, about 55%, about 60%, about 67% of the weight of the sustained release portion.

It should be understood that when a formulation comprises a sustained release portion or an immediate release portion which are morphologically separated, the content of the material should be calculated based on the portion where the material is located. For example, the coated tablet or osmotic pump tablet can comprise a sustained release tablet core and/or a sustained coating. When the tablet core or coating comprises a material such as a film-forming material or a matrix material, the content should be calculated based on the corresponding tablet core or coating.

In an embodiment, the immediate release portion comprises a film-forming material, the film-forming material is about 50%-70%, for example about 50%, about 55%, about 60%, about 65%, about 70% of the weight of the immediate release portion.

In an embodiment, the formulation of the present disclosure, in the sustained release portion, the immediate release portion, or another portion beyond them, can also optionally comprise one or more of the following pharmaceutically acceptable excipients: a binder, a filler, a disintegrant, a lubricant. The amount of these pharmaceutically acceptable excipients can be selected by those skilled in the art according to needs.

The following are examples of some pharmaceutically acceptable excipients, without limitation:
The binder can be one or more selected from the group consisting of polyethylene glycol, starch, hydroxypropyl cellulose, hydroxypropyl methyl cellulose, pregelatinized starch, polyvinylpyrrolidone, gum arabic and gelatin, preferably hydroxypropyl cellulose.

The filler can be one or more selected from the group consisting of starch, pregelatinized starch, modified starch, microcrystalline cellulose, silicified microcrystalline cellulose, low-substituted hydroxypropyl cellulose glucose, sucrose, lactose, sorbitol, mannitol, erythritol, calcium carbonate and calcium hydrophosphate, preferably mannitol, microcrystalline cellulose and silicified microcrystalline cellulose, preferably microcrystalline cellulose, lactose or mannitol.

The disintegrant can be one or more selected from the group consisting of microcrystalline cellulose, carboxymethyl cellulose, copovidone, croscarmellose sodium, calcium carboxymethylcellulose, sodium carboxymethyl starch, low-substituted hydroxypropyl cellulose, hydroxymethyl starch, alginic acid, sodium alginate, guar gum, corn starch and magnesium aluminum silicate, preferably copovidone.

The lubricant can be one or more selected from the group consisting of magnesium stearate, stearic acid, stearate, sodium stearyl fumarate, sodium lauryl sulfate, polyethylene glycol, sodium benzoate, sucrose fatty acid ester, micronized silica gel, talcum powder, glyceryl monostearate, glyceryl behenate, glyceryl palmitostearate, stearic acid and hydrogenated vegetable oil, preferably magnesium stearate or glyceryl behenate.

In another aspect, provided is a pharmaceutical composition comprising the solid formulation of the present disclosure. The pharmaceutical composition can also comprise one or more pharmaceutically acceptable auxiliaries.

### Release of the formulation of the disclosure

By including an immediate release portion and a sustained release portion, the formulation of the present disclosure can exhibit a combination of immediate release and sustained release dual release mode, enabling the rapid onset of the pharmaceutical ingredient while providing an effective and stable blood concentration for a longer period of time.

In an embodiment, based on the total weight of the active ingredient in the solid formulation, the active ingredient is released in an amount of about 5%-50% within 15 min, for example about 5%, about 10%, about 15%, about 20%, about 25%, about 30%, about 45% or about 50%. In a preferable embodiment, based on the total weight of the active ingredient in the solid formulation, the active ingredient is released in an amount of about 10%-30% within 15 min, for example about 10%, about 15%, about 20%, about 25% or about 30%, particularly about 20%.

In an embodiment of the present formulation, the active ingredient in the immediate release portion has a dissolution rate within 15 min of about 0.03-0.37 mg/min, for example, about 0.07 mg/min, about 0.13 mg/min, about 0.15 mg/min, about 0.17 mg/min, about 0.21 mg/min, about 0.24 mg/min, about 0.28 mg/min or about 0.33 mg/min. In a preferable embodiment of the present formulation, the active ingredient in the immediate release portion has a dissolution rate within 15 min of about 0.07-0.22 mg/min, for example, about 0.07 mg/min, about 0.13 mg/min, about 0.15 mg/min, about 0.17 mg/min or about 0.21 mg/min, specifically preferably about 0.15 mg/min.

In another embodiment of the present formulation, the ratio of the average dissolution rate of the active ingredient in the immediate release portion within 15 min to the average dissolution rate of the active ingredient in the sustained release portion over the entire release time range is about 0.5:1-100:1, for example about 1.3:1, about 10.4:1, about 30:1, about 64.5:1 or about 96.5:1. In a preferable embodiment, the ratio is about 1.4:1-11:1, for example about 1.6:1, about 3.3:1, about 6.7:1, about 8.5:1 or 10:1. In a specifically preferable embodiment, the ratio is about 3:1-6:1, for example about 3.3:1, about 3.9:1, about 4.5:1, about 5.2:1 or about 5.8:1.

The dissolution rate and release amount can be calculated from the dissolution-time curve of the active ingredient. The dissolution can be determined by conventional methods in the field, for example, according to the USP II method (Paddle Method) of United States Pharmacopeia.

In an embodiment, the active ingredient in the formulation according to the present disclosure has a total release amount within 1 h of about 20%-60%, for example about 20%, about 25%, about 30%, about 35%, about 40%, about 45%, about 50%, about 55% or about 60%. In a preferable embodiment, the release amount is about 30%-50%, for example about 30%, about 35%, about 40%, about 45% or about 50%.

In an embodiment, the active ingredient in the formulation according to the present disclosure has a total release amount within 3 h of about 55%-95%, for example about 55%, about 60%, about 65%, about 70%, about 75%, about 80%, about 85%, about 90% or about 95%. In a preferable embodiment, the release amount is about 60%-80%, for example about 60%, about 65%, about 70%, about 75% or about 80%.

In an embodiment, the active ingredient in the formulation according to the present disclosure has a total release amount within 5 h of no less than about 80%, for example about 80%, about 85%, about 90%, about 95% or about 99% or higher. In a preferable embodiment, the release amount is no less than about 85%, for example about 85%, about 90%, about 95%, about 96% or about 97% or higher.

In an embodiment, the active ingredient in the formulation according to the present disclosure has a total release amount within 15 min of about 5%-50%, the total release amount within 1 h is about 20%-65%; the total release amount within 3 h is about 55%-90%; and the total release amount within 5 h is no less than about 80%.

The calculations of above-mentioned release amount are based on the total amount of active ingredient in the formulation prior to release.

In an embodiment, after a single administration, the time required by the formulation according to the present disclosure for achieving a blood concentration of 17 ng/ml is about 10-60 min, for example about 15 min, about 20 min, about 25 min, about 30 min, about 35 min, about 40 min, about 45 min, about 50 min, about 55 min or about 60 min. In a preferable embodiment, after a single administration, the time required by the formulation according to the present disclosure for achieving a blood concentration of 17 ng/ml is about 25-45 min, for example about 25 min, about 30 min, about 35 min, about 40 min, about 45 min or about 50 min. In a specifically preferable embodiment, after a single administration, the time required by the formulation according to the present disclosure for achieving a blood concentration of 17 ng/ml is about 20-30 min, for example about 20 min, about 22 min, about 24 min, about 26 min, about 28 min or about 30 min.

In an embodiment, after a single administration, the duration time of the solid formulation according to the present disclosure for achieving a blood concentration over 17 ng/ml is about 8-12 h (for example about 8, about 8.5, about 9, about 9.5, about 10, about 10.5, about 11, about 11.5 or about 12 h), preferably about 9-11 h.

The blood concentration of 17 ng/ml herein corresponds to the half inhibitory concentration of JAK1/3 *in vitro* (i.e., IC₅₀ value).

As used herein, "single administration" refers to a solid formulation of one dosage unit (for example, one tablet or one pill) containing 11 mg or 22 mg of the active ingredient (e.g., Tofacitinib citrate) for one administration. The administration frequency can be, for example, once daily. The administration subject can be a mammal, for example a human.

In a preferrable embodiment of the formulation herein, the active ingredient can be Tofacitinib or a pharmaceutically acceptable salt thereof, particularly Tofacitinib citrate.

### Preparation process according to the present disclosure

Provided is also a process for preparing the solid formulation according to the present disclosure, comprising
1) combining the active ingredient with one or more pharmaceutically acceptable excipients to give the immediate release portion,
2) combining the active ingredient with one or more pharmaceutically acceptable excipients to give the sustained release portion,
3) combing the immediate release portion and the sustained release portion to give the solid formulation;
wherein
step 1) and step 2) are conducted in any order independently;
the active ingredients and the excipients in step 1) and step 2) are used independently.

In an embodiment, the active ingredients in step 1) and step 2) are each independently Tofacitinib citrate.

In another embodiment, the total amount of the active ingredients in step 1) and step 2) is 100%. In other words, the active ingredient in the formulation is only present in the immediate release portion and sustained release portion.

The immediate release portion, sustained release portion, active ingredient, excipient in the formulation of the present disclosure can be referred to the corresponding descriptions above.

In an exemplary embodiment, a pressing method can be used, comprising:
1. Preparing the immediate release layer granules: Mixing the active ingredient, the filler, the disintegrant and half of the lubricant according to the metering ratio uniformly; using a dry granulator to prepare the above mixture into granules, and then mixing them with the remaining half of the lubricant to give the blended granules of the immediate release layer.
2. Preparing the sustained release layer granules: Mixing the active ingredient, the filler, the sustained release matrix material and half of the lubricant according to the metering ratio uniformly; using a dry granulator to prepare the above mixture into granules, and then mixing them with the remaining half of the lubricant to give the blended granule of the sustained release layer.
3. Compression: Pressing the immediate release layer blended granules and the sustained release layer blended granules into a bilayer tablet by using a bilayer tableting machine to give the final bilayer tablet.
4. Coating: If desired, adding a protective coating commonly used in the art. The coating may not contain the active ingredient.

In an embodiment, the process for preparing a coated tablet is to press a sustained release tablet, and then apply an immediate release coating containing the active ingredient. Such a process for preparing the coated tablet comprises the following steps:
1. Preparing sustained release granules: Mixing the active ingredient, the filler, the sustained release matrix material and half of the lubricant according to the metering ratio uniformly; using a dry granulator to prepare the above mixture into granules and mix them with the remaining half of the lubricant to give the blended granules.
2. Compression: Pressing the above blended granules into a sustained release tablet using a tableting machine.
3. Coating: Mixing the active ingredient, the filler and the membrane uniformly, which are added into an isopropanol solution containing 10 wt% purified water with sufficiently and uniformly stirring and prepared to be a suspension with a solid content of about 7%. Spraying the above suspension coating on the sustained release tablet, which after drying is prepared into a coated tablet.

In an embodiment, the process for preparing a composition containing the active ingredient is to prepare immediate release pellets by extrusion spheronization, coating a part of the pellets into sustained release pellets, and filling both of them into a capsule in proportion. Such a process for preparing a capsule comprises the following steps:
1. Preparing the immediate release pellets: Mixing the active ingredient, the filler, the binder and the lubricant according to the metering ratio uniformly, to which is added an appropriate amount of purified water to prepare a soft material, extruding the above soft material using an extruding spheronizer into wet drug-containing immediate release pellets, and then drying the above pellets using a fluidized bed to give immediate release pellets.
2. Preparing sustained release pellets: Adding the sustained coating membrane and the filler into an isopropanol solution containing 10 wt% purified water with sufficiently and uniformly stirring which is prepared into a suspension with a solid content of about 7%. Placing a part of the immediate release pellets according to the metering ratio into a fluidized bed, coating the above solution on the surface of the immediate release pellets, and preparing the sustained release pellet after drying.
3. Capsules filling: filling the immediate release pellets and the sustained release pellets into capsules using a capsule filling machine according to the respective weights to give capsules.

### Pharmaceutical method and pharmaceutical use

Provided is the solid formulation or the pharmaceutical composition according to the present disclosure for use in the treatment or prevention of a disease comprising lupus, multiple sclerosis, rheumatoid arthritis, psoriatic arthritis, type I diabetes and diabetic complications, cancer, asthma, atopic dermatitis, autoimmune thyroid disease, moderate-to-severe active ulcerative colitis, Crohns' disease, ankylosing spondylitis, juvenile idiopathic arthritis, immune system disorder baldness, vitiligo, systemic lupus erythematosus, Alzheimer's disease and leukemia.

### Beneficial effect

Compared with the prior art, the solid formulation of the present disclosure comprises an immediate release portion and a sustained release portion. On the basis that the immediate release achieves the potency, it also has a sustained release effect, such that the potency can rapidly onset and be maintained for a long time.

In particular, in the coated tablet and osmotic pump tablet of the present disclosure, the tablet core is the sustained release portion, and the outer coating also contains a certain amount of active ingredient. As the immediate release portion of the dual-release formulation, the release rate of the active ingredient in the early stage is accelerated, eliminating the delayed release phenomenon in the prior art. Meanwhile, the sustained release portion can achieve stable blood concentration for a long time.

Further, the immediate release portion of the formulation of the present disclosure has a faster release rate, where within 15 min, the dissolution rate of the active ingredient (e.g., Tofacitinib or a pharmaceutically acceptable salt thereof) can reach about 0.03 mg/min-0.37 mg/min. The dissolution rate of the active ingredient can be changed by adjusting the contents of the active ingredient (e.g., Tofacitinib or a pharmaceutically acceptable salt thereof) in the immediate release portion and the active ingredient (e.g., Tofacitinib or a pharmaceutically acceptable salt thereof) in the sustained release portion to meet the requirements of different medication environments and patients with different conditions.

In another aspect, in the formulation of the present disclosure, the dissolution rate ratio of the active ingredient (e.g., Tofacitinib or a pharmaceutically acceptable salt thereof) in the immediate release portion and the active ingredient (e.g., Tofacitinib or a pharmaceutically acceptable salt thereof) in the sustained release portion within 15 min is about 0.5:1-100:1. The dissolution rate ratio can be changed by adjusting the contents of the active ingredient in immediate release portion and the active ingredient in the sustained release portion to achieve immediate release or sustained release of the active ingredient to meet the requirements of different medication environments and patients with different conditions.

In addition, the formulation of the present disclosure can provide longer duration of single administration blood concentration above 17 ng/ml, for example about 9-11 h. Compared to commercial formulations, the duration is longer, thereby allowing the drug to remain stable concentration fluctuations in the body so as to achieve dosing once daily and provide fast and durable disease management.

### Examples

The technical solutions of the present disclosure will be further described below by reference to specific examples. It should be noted that the examples are exemplary only, rather than any limitation to the protection scope of present disclosure. The present disclosure can also have other embodiments or be practiced with various means. Unless otherwise stated, all percentages, parts, ratios or the like herein are provided on a weight basis. Unless otherwise stated, the apparatus, reagents, raw materials or the like herein are commercially available.

The sources of some of the materials used are listed below: Tofacitinib citrate, purchased from Haiyou Freda; glyceryl behenate, purchased from GATTEFOSSE, Compritol ATO888; hydroxyethyl cellulose, purchased from Ashland, Natrosol HX; hydroxypropyl methyl cellulose, purchased from Shin-Etsu, Metolose SR 90SH; cellulose acetate, purchased from Eastman, CA-398-10.

In the following examples, the formulations were tested for dissolution by the USP II method (Paddle Method) of United States Pharmacopeia. The specific conditions were as follows: phosphate buffer with pH 6.8 was used as the dissolution medium, the volume of the dissolution medium was 900 ml, the rotational speed of the dissolution assay was 50 rpm, the assay temperature was 37 °C.

The parameters of the dissolution assay method used are as follows.

| | |
|---|---|
| Dissolution method | USP II method (Paddle Method) |
| Dissolution medium | phosphate buffer, pH 6.8 |
| medium volume | 900 ml |
| Rotary speed | 50 rpm |
| Temperature | 37°C |

Dissolution sample analysis: The solution obtained in the dissolution assay was filtered with a 0.45 µm filtration membrane to collect the filtrate, which was measured by high performance liquid chromatography at a wavelength of 210 nm with Agilent 1200.

### Example 1. Preparation of bilayer tablets

### 1. Preparation:

Preparation was conducted according to the specific compositions and amounts (parts by weight, hereafter) in Table 1.

Immediate release layer granules: According to the metering ratio, Tofacitinib, the filler, the disintegrant and half amount of lubricant were mixed uniformly using a hopper mixer (Nantong BEITE HBD-200); the above mixture was prepared into granules using a dry granulator (Alexanderwerk WP120, Germany), which were mixed with the remaining half amount of lubricant using a hopper mixer (Nantong BEITE HBD-200) to give the blended granules of the immediate release layer.

Sustained release layer granule: According to the metering ratio, Tofacitinib, the filler, the sustained release matrix material and half amount of lubricant were mixed uniformly using a hopper mixer (Nantong BEITE HBD-200); the above mixture was prepared into granules using a dry granulator (Alexanderwerk WP120, Germany), which were mixed with the remaining half amount of the lubricant using a hopper mixer (Nantong BEITE HBD-200) to give the blended granules of the sustained release layer.

Compression: The immediate release layer blended granules and the sustained release layer blended granules were pressed into bilayer tablets using a bilayer tableting machine (FETTE P3030) to give the final bilayer tablets.

According to the above procedure, the bilayer tablets 1-1 to 1-10 of the present disclosure were prepared.

**Table 1 Specific composition of bilayer tablets (parts by weight)**

| Component | Function | bilayer tablet 1-1 | bilayer tablet 1-2 | bilayer tablet 1-3 | bilayer tablet 1-4 | bilayer tablet 1-5 | bilayer tablet 1-6 | bilayer tablet 1-7 | bilayer tablet 1-8 | bilayer tablet 1-9 | bilayer tablet 1-10 |
|---|---|---|---|---|---|---|---|---|---|---|---|
| Immediate release layer | | | | | | | | | | | |
| Tofacitinib citrate | active ingredien | 0.5 | 1 | 2 | 3 | 4 | 5 | 2 | 2 | 2 | 2 |
| microcrystalline cellulose | filler | 138.2 | 138 | 137.5 | 137 | 136.5 | 136 | 143.5 | 127.5 | 117.5 | 107.5 |
| lactose | filler | 138.3 | 138 | 137.5 | 137 | 136.5 | 136 | 143.5 | 127.5 | 117.5 | 107.5 |
| copovidone | disintegrant | 20 | 20 | 20 | 20 | 20 | 20 | 8 | 40 | 60 | 80 |
| magnesium stearate | lubricant | 3 | 3 | 3 | 3 | 3 | 3 | 3 | 3 | 3 | 3 |

| Sustained release layer | | | | | | | | | | | |
|---|---|---|---|---|---|---|---|---|---|---|---|
| Tofacitinib citrate | active ingredien | 9.5 | 9 | 8 | 7 | 6 | 5 | 8 | 8 | 8 | 8 |
| microcrystalline cellulose | filler | 93.2 | 93.5 | 94 | 94.5 | 95 | 95.5 | 114 | 94 | 74 | 34 |
| lactose | filler | 93.3 | 93.5 | 94 | 94.5 | 95 | 95.5 | 114 | 94 | 74 | 34 |
| glyceryl behenate | sustained release matrix material | 200 | 200 | 200 | 200 | 200 | 200 | 160 | 200 | 240 | 320 |
| magnesium stearate | lubricant | 4 | 4 | 4 | 4 | 4 | 4 | 4 | 4 | 4 | 4 |

### 2. Dissolution assay of the formulations

According to the procedure described above, the dissolutions of each bilayer tablets in phosphate buffer, pH 6.8 were determined, and the results were shown in Table 2.

**Table 2 Dissolutions of bilayer tablets**

| | Dissolution (%) | | | | | | | |
|---|---|---|---|---|---|---|---|---|
| Time point (h) | 0.25 | 1 | 2 | 3 | 4 | 5 | 6 | 7 |
| bilayer tablet 1-1 | 5 | 27 | 45 | 58 | 69 | 80 | 89 | 96 |
| bilayer tablet 1-2 | 10 | 31 | 48 | 60 | 71 | 81 | 90 | 96 |
| bilayer tablet 1-3 | 20 | 39 | 54 | 65 | 74 | 83 | 91 | 97 |
| bilayer tablet 1-4 | 30 | 46 | 59 | 69 | 77 | 85 | 92 | 99 |
| bilayer tablet 1-5 | 40 | 54 | 65 | 73 | 80 | 87 | 93 | 99 |
| bilayer tablet 1-6 | 50 | 62 | 71 | 78 | 84 | 89 | 94 | 100 |
| bilayer tablet 1-7 | 20 | 42 | 64 | 82 | 99 | 99 | 100 | 100 |
| bilayer tablet 1-8 | 20 | 37 | 50 | 61 | 72 | 83 | 92 | 99 |
| bilayer tablet 1-9 | 20 | 33 | 45 | 54 | 63 | 72 | 81 | 90 |
| bilayer tablet 1-10 | 20 | 27 | 37 | 46 | 52 | 59 | 65 | 68 |

From the data in Table 2, it can be seen that the bilayer tablets prepared with different compositions had wide drug release ranges. The release amount in 15 min was the content of the active ingredient Tofacitinib in the immediate release layer. The results showed that the immediate release layers with different compositions released the drugs within 15 min completely. In the subsequent process, the solid formulations can also continuously release the active ingredient Tofacitinib at a steady rate, providing effective and stable blood concentrations for a longer time. In particular, the release of formulations 1-1 to 1-8 was completed within 7 h, showing a more advantageous release effect.

### Example 2 Preparation of coated tablets

### 1. Preparation:

Preparation was conducted according to the specific compositions and amounts (parts by weight, hereafter) in Table 3.

Sustained release granule: According to the metering ratio, Tofacitinib, the filler, the sustained release matrix material and half amount of lubricant were mixed uniformly using a hopper mixer (Nantong BEITE HBD-200); the above mixture was prepared into granules using a dry granulator (Alexanderwerk WP120, Germany), which were mixed with the remaining half amount of lubricant using a hopper mixer (Nantong BEITE HBD-200) to give the blended granules.

Compression: The above blended granules were pressed into sustained release tablets using a tableting machine (FETTE P2020).

Coating: Tofacitinib and membrane material were mixed uniformly, then added to an isopropanol solution containing 10 wt% purified water with sufficiently and uniformly stirring and prepared to be a suspension with a solid content of about 7%. The above suspension was sprayed onto sustained release tablets using a coating pan (O'Hara Lab coat M), which were dried to give coated tablets. During the spraying process, the material temperature was 25-35°C, and the inlet air volume was maintained as 50-100 cm³/h. After the spraying was completed, the coated tablets of Tofacitinib were dried in a 30°C coating machine for 2 h, until the loss on drying was less than 2.0%, to give the final coated tablets.

According to the above procedure, the coated tablets 1-1 to 1-10 of the present disclosure were prepared.

**Table 3 Specific composition of coated tablets (parts by weight)**

| Component | Function | coated tablet 1-1 | coated tablet 1-2 | coated tablet 1-3 | coated tablet 1-4 | coated tablet 1-5 | coated tablet 1-6 | coated tablet 1-7 | coated tablet 1-8 | coated tablet 1-9 | coated tablet 1-10 |
|---|---|---|---|---|---|---|---|---|---|---|---|
| Sustained release tablet core | | | | | | | | | | | |
| Tofacitinib citrate | active ingredient | 9.5 | 9 | 8 | 7 | 6 | 5 | 8 | 8 | 8 | 8 |
| microcrystalline cellulose | filler | 93.2 | 93.5 | 94 | 94.5 | 95 | 95.5 | 114 | 94 | 74 | 34 |
| lactose | filler | 93.3 | 93.5 | 94 | 94.5 | 95 | 95.5 | 114 | 94 | 74 | 34 |
| glyceryl behenate | sustained release matrix material | 200 | 200 | 200 | 200 | 200 | 200 | 160 | 200 | 240 | 320 |
| magnesium stearate | lubricant | 4 | 4 | 4 | 4 | 4 | 4 | 4 | 4 | 4 | 4 |

| Coating prescription | | | | | | | | | | | |
|---|---|---|---|---|---|---|---|---|---|---|---|
| Tofacitinib citrate | active ingredient | 0.5 | 1 | 2 | 3 | 4 | 5 | 2 | 2 | 2 | 2 |
| ethyl cellulose | membrane material | 10 | 10 | 10 | 10 | 10 | 10 | 4 | 20 | 30 | 40 |
| mannitol | filler | 10 | 10 | 10 | 10 | 10 | 10 | 4 | 20 | 30 | 40 |
| 90% isopropanol* | solvent | appropriate amount | appropriate amount | appropriate amount | appropriate amount | appropriate amount | appropriate amount | appropriate amount | appropriate amount | appropriate amount | appropriate amount |

| | | | | | | | | | | | |
|---|---|---|---|---|---|---|---|---|---|---|---|
| * volatilized during preparation. | | | | | | | | | | | |

### 2. Dissolution assay of the formulations

According to the procedure described above, the dissolutions of each coated tablets in phosphate buffer, pH 6.8 were determined, and the results were shown in Table 4.

**Table 4 Dissolutions of coated tablets**

| | Dissolution (%) | | | | | | | |
|---|---|---|---|---|---|---|---|---|
| Time point (h) | 0.25 | 1 | 2 | 3 | 4 | 5 | 6 | 7 |
| coated tablet 1-1 | 5 | 20 | 40 | 55 | 69 | 80 | 89 | 96 |
| coated tablet 1-2 | 10 | 24 | 43 | 57 | 71 | 81 | 90 | 96 |
| coated tablet 1-3 | 20 | 33 | 49 | 62 | 74 | 83 | 91 | 97 |
| coated tablet 1-4 | 30 | 41 | 56 | 67 | 77 | 85 | 92 | 97 |
| coated tablet 1-5 | 40 | 49 | 62 | 72 | 80 | 87 | 93 | 97 |
| coated tablet 1-6 | 50 | 58 | 68 | 76 | 84 | 89 | 94 | 98 |
| coated tablet 1-7 | 20 | 40 | 59 | 76 | 92 | 98 | 99 | 99 |
| coated tablet 1-8 | 20 | 30 | 45 | 57 | 69 | 80 | 90 | 98 |
| coated tablet 1-9 | 20 | 30 | 40 | 51 | 61 | 70 | 79 | 88 |
| coated tablet 1-10 | 20 | 26 | 36 | 44 | 50 | 57 | 64 | 66 |

From the data in Table 4, it can be seen that the coated tablets prepared with different compositions had wide drug release ranges. The release amount in 15 min was the content of the active ingredient Tofacitinib in the immediate release layer. The results showed that the coatings with different compositions released the active ingredient Tofacitinib within 15 min completely. In the subsequent process, the solid formulations can also continuously release the active ingredient Tofacitinib at a steady rate, providing effective and stable blood concentrations for a longer time. In particular, the release of formulations 1-1 to 1-8 was completed within 7 h, showing a more advantageous release effect.

### Example 3 Preparation of osmotic pump tablets

### 1. Preparation:

Preparation was conducted according to the specific compositions and amounts (parts by weight, hereafter) in Table 5.

Tablet core granule: According to the metering ratio, Tofacitinib, the filler, the sustained release matrix material and half amount of lubricant were mixed uniformly using a hopper mixer (Nantong BEITE HBD-200); the above mixture was prepared into granules using a dry granulator (Alexanderwerk WP120, Germany), which were mixed with the remaining half amount of lubricant using a hopper mixer (Nantong BEITE HBD-200) to give the blended granules.

Compression: The above blended granules were pressed into tablet cores using a tableting machine (FETTE P2020).

Coating: Tofacitinib and membrane material were mixed uniformly, then added to an isopropanol solution containing 10 wt% purified water with sufficiently and uniformly stirring and prepared to be a suspension with a solid content of about 7%. The above suspension was sprayed onto tablet cores using a coating pan (O'Hara Lab coat M), which were dried to give semipermeable membrane coated tablets. During the spraying process, the material temperature was 25-35°C, and the inlet air volume was maintained as 50-100 cm³/h. After the spraying was completed, the semipermeable membrane coated tablets of Tofacitinib were dried in a 30°C coating machine for 2 h, until the loss on drying was less than 2.0%.

Punching: The above semipermeable membrane coated tablets were punched by a mechanical puncher (BEKING Surom) with a 1.0 mm drill bit to give the final osmotic pump tablets.

According to the above procedure, the osmotic pump tablets 1-1 to 1-10 of the present disclosure were prepared.

**Table 5 Specific composition of osmotic pump tablets (parts by weight)**

| Component | Funcntion | osmotic pump tablet 1-1 | osmotic pump tablet 1-2 | osmotic pump tablet 1-3 | osmotic pump tablet 1-4 | osmotic pump tablet 1-5 | osmotic pump tablet 1-6 | osmotic pump tablet 1-7 | osmotic pump tablet 1-8 | osmotic pump tablet 1-9 | osmotic pump tablet 1-10 |
|---|---|---|---|---|---|---|---|---|---|---|---|
| Sustained release tablet core | | | | | | | | | | | |
| Tofacitinib citrate | Active Ingredient | 9.5 | 9 | 8 | 7 | 6 | 5 | 8 | 8 | 8 | 8 |
| microcrystalline cellulose | filler | 93.2 | 93.5 | 94 | 94.5 | 95 | 95.5 | 114 | 94 | 74 | 34 |
| lactose | filler | 93.3 | 93.5 | 94 | 94.5 | 95 | 95.5 | 114 | 94 | 74 | 34 |
| hydroxyethyl cellulose | sustained release matrixma terial | 200 | 200 | 200 | 200 | 200 | 200 | 160 | 200 | 240 | 320 |
| magnesiu m stearate | lubricant | 4 | 4 | 4 | 4 | 4 | 4 | 4 | 4 | 4 | 4 |

| Coating prescription | | | | | | | | | | | |
|---|---|---|---|---|---|---|---|---|---|---|---|
| Tofacitinib citrate | Active Ingredient | 0.5 | 1 | 2 | 3 | 4 | 5 | 2 | 2 | 2 | 2 |
| cellulose acetate | membrane material | 10 | 10 | 10 | 10 | 10 | 10 | 4 | 20 | 30 | 40 |
| mannitol | filler | 5 | 5 | 5 | 5 | 5 | 5 | 2 | 10 | 15 | 20 |
| 90% isopropano l* | solvent | appropr iate amount | appropr iate amount | appropr iate amount | appropr iate amount | appropr iate amount | appropr iate amount | appropr iate amount | appropr iate amount | appropr iate amount | appropr iate amount |

| | | | | | | | | | | | |
|---|---|---|---|---|---|---|---|---|---|---|---|
| * volatilized during preparation. | | | | | | | | | | | |

### 2. Dissolution assay of the formulations

According to the procedure described above, the dissolutions of each osmotic pump tablets in phosphate buffer, pH 6.8 were determined, and the results were shown in Table 6.

**Table 6 Dissolutions of osmotic pump tablets**

| | Dissolution (%) | | | | | | | |
|---|---|---|---|---|---|---|---|---|
| Time point (h) | 0.25 | 1 | 2 | 3 | 4 | 5 | 6 | 7 |
| osmotic pump tablet 1-1 | 5 | 9 | 40 | 66 | 84 | 89 | 97 | 97 |
| osmotic pump tablet 1-2 | 10 | 14 | 43 | 68 | 85 | 90 | 97 | 97 |
| osmotic pump tablet 1-3 | 20 | 22 | 49 | 72 | 87 | 91 | 97 | 97 |
| osmotic pump tablet 1-4 | 30 | 32 | 56 | 75 | 88 | 92 | 98 | 98 |
| osmotic pump tablet 1-5 | 40 | 43 | 62 | 79 | 90 | 93 | 99 | 99 |
| osmotic pump tablet 1-6 | 50 | 52 | 68 | 82 | 92 | 94 | 99 | 99 |
| osmotic pump tablet 1-7 | 20 | 55 | 78 | 93 | 99 | 99 | 99 | 99 |
| osmotic pump tablet 1-8 | 20 | 20 | 44 | 67 | 82 | 91 | 97 | 99 |
| osmotic pump tablet 1-9 | 20 | 20 | 37 | 52 | 65 | 75 | 80 | 82 |
| osmotic pump tablet 1-10 | 20 | 20 | 32 | 40 | 45 | 46 | 47 | 47 |

From the data in Table 6, it can be seen that the osmotic pump tablet prepared with different compositions had wide drug release ranges. The release amount in 15 min (0.25 h) was the content of the active ingredient Tofacitinib in the semipermeable membrane coating. The results showed that the coatings with different compositions released the active ingredient Tofacitinib within 15 min completely. In the subsequent process, the solid formulations can also continuously release the active ingredient Tofacitinib at a steady rate, providing effective and stable blood concentrations for a longer time. In particular, the release of formulations 1-1 to 1-8 was completed within 7 h, showing a more advantageous release effect.

### Example 4 Comparison of dissolutions

According to the above procedure, the dissolutions of Tofacitinib formulations among different dosage forms (20% for the immediate release portion of Tofacitinib and 80% for the sustained release portion of Tofacitinib) were determined for comparison, where the dissolution of each formulation was that in phosphate buffer, pH 6.8. The results were shown in Table 7 below.

**Table 7 Dissolutions of formulations**

| | Dissolution (%) | | | | | | | |
|---|---|---|---|---|---|---|---|---|
| Time point (h) | 0.25 | 1 | 2 | 3 | 4 | 5 | 6 | 7 |
| bilayer tablet 1-3 | 20 | 39 | 54 | 65 | 74 | 83 | 91 | 97 |
| coated tablet 1-3 | 20 | 33 | 49 | 62 | 74 | 83 | 91 | 97 |
| osmotic pump tablet 1-3 | 20 | 22 | 49 | 72 | 87 | 91 | 97 | 97 |

From the data in the table above, it can be seen that the above three different dosage forms of Tofacitinib formulations all have a special release pattern of immediate release in the first 15 min and then sustained release for more than 5 h. Such release pattern not only maintains the rapid onset of action after administration in the early stage, but also maintains the clinical effect for a long time, providing more stable blood concentrations.

### Example 5 Pharmacokinetic Studies

The coated tablets1-3 prepared in Example 2 were used.

### 1. Testing formulations

Testing formulation T: Tofacitinib coated tablets were prepared according to the compositions and procedure of coated tablets 1-3 in Example 2, with the strength of 11 mg, once a day.

Reference formulation R: commercial Tofacitinib sustained release tablet (Xeljanz XR), with the strength of 11 mg, once a day.

### 2. Testing procedure

### 1) Single administration test

An open, randomized, double-period, double-crossover self-controlled trial design method (7-day washout period) was used, where10 healthy male subjects were randomly divided into 2 groups where 5 in each group. The grouping design was shown in the table below:

**Table 8 Grouping design**

| Period | Group 1 | Group 2 |
|---|---|---|
| Period I | T | R |
| Period II | R | T |

The subjects entered the Phase I clinical trial ward before the testing day, were given a uniform light diet in the evening, and then fasted overnight (at least 10 h, free for getting water). The subjects were orally given 11 mg of Tofacitinib coated tablets with empty stomach at about 8 am the next day and were given 240 ml of water after 10 min. Alternatively, the subjects were orally given 11 mg of Tofacitinib sustained release tablets with empty stomach at about 8 am and 8 pm the next day, respectively along with 240 ml of water. No water was allowed for 2 h after administration with the upper body upright. The lunches were given after 4 h.

Blood sample collection procedure: 0 h before administration (within 1 h before administration) and 0.5, 1, 1.5, 2, 2.5, 3, 3.5, 4, 4.5, 5, 6, 7, 8, 12, 24, 36 h after administration (17 blood collection points), 4 mL of cubital venous blood was collected, and the blood samples were placed in blood collection tubes anticoagulated with heparin sodium. After collection, the samples were centrifuged at 3500 rpm for 5 min at 4°C. The plasma was divided into two parts, 1 mL of plasma was added to the formal tube and the remaining plasma was added to the backup tube. The samples were stored in a -20°C refrigerator 2 h after collection for subsequent use.

The LC-MS/MS method was used to determine the concentration of Tofacitinib in each plasma sample. The pharmacokinetic statistical software DAS 2.0 was used for calculation and biostatistical analysis was performed. The test results were shown in Table 9 and Figure 1.

From the blood concentration curve, it can be seen that the time for the reference sustained release tablet to reach 17 ng/ml was about 90 min. In contrast, the time for the coated tablet of the present disclosure to reach 17 mg/ml was about 30 min.

**Table 9 Main pharmacokinetic parameters of Tofacitinib single administration**

| | Tofacitinib sustained release tablet | Tofacitinib coated tablet (T) |
|---|---|---|
| | Commercial (R) | |
| Dosage form | Sustained release tablet | Coated tablet (Sustained release) |
| Administration Route | oral | oral |
| Dosage | 11 mg, Once a day | 11 mg, Once a day |
| Cₘₐₓ (ng/mL) | 65±28 | 57±25 |
| AUC₀₋₃₆ₕ (ng·h/ml) | 367±116 | 414±120 |
| Tₘₐₓ (h) | 3.0 [2.5~4.0] | 2.1[1.2~3.4] |
| Ta (h) | 7.6 | 10.1 |

Ta refers to the time that the blood concentration can be maintained above 17 ng/mL (IC₅₀).

It can be seen from Table 9 that the maximum blood concentration Cₘₐₓ of the Tofacitinib coated tablet of the present disclosure was significantly lower than that of the commercially available sustained release tablet, where the fluctuation was more stable than that of the commercially available sustained release tablet, and the area under the curve of blood concentration was significantly increased. The Ta value was extended by about 33% as compared to that of Tofacitinib commercial sustained release tablet.

### Example 6 Pharmacokinetic Studies of Tofacitinib formulation

The coated tablets 1-2, 1-3 and 1-6 prepared in Example 2 were used.

### 1. Testing formulations

Testing formulation T1: Tofacitinib coated tablet prepared according to the composition and procedure of coated tablet 1-2 in Example 2 with strength of 11 mg, once a day.

Testing formulation T2: Tofacitinib coated tablet prepared according to the composition and procedure of coated tablet 1-3 in Example 2 with strength of 11 mg, once a day.

Testing formulation T3: Tofacitinib coated tablet prepared according to the composition and procedure of coated tablet 1-6 in Example 2 with strength of 11 mg, once a day.

### 2. Testing procedure

### 1) Single administration test

An open, randomized, double-period, triple-crossover self-controlled trial design method (7-day washout period) was used, where 9 healthy male subjects were randomly divided into 3 groups where 3 in each group. The grouping design was shown in the table below:

**Table 10 Grouping design**

| Period | Group 1 | Group 2 | Group 3 |
|---|---|---|---|
| Period I | T1 | T3 | T2 |
| Period II | T2 | T1 | T3 |
| Period III | T3 | T2 | T1 |

The subjects entered the Phase I clinical trial ward before the testing day, were given a uniform light diet in the evening, and then fasted overnight (at least 10 h, free for getting water). The subjects were orally given 11 mg of Tofacitinib coated tablets with empty stomach at about 8 am the next day and were given 240 ml of water after 10 min. No water was allowed for 2 h after administration with the upper body upright. The lunches were given after 4 h.

Blood sample collection procedure: 0 h before administration (within 1 h before administration) and 0.5, 1, 1.5, 2, 2.5, 3, 3.5, 4, 4.5, 5, 6, 7, 8, 12, 24, 36 h after administration (17 blood collection points), 4 mL of cubital venous blood was collected, and the blood samples were placed in blood collection tubes anticoagulated with heparin sodium. After collection, the samples were centrifuged at 3500 rpm for 5 min at 4°C. The plasma was divided into two parts, 1 mL of plasma was added to the formal tube and the remaining plasma was added to the backup tube. The samples were stored in a -20 °C refrigerator 2 h after collection for subsequent use.

The LC-MS/MS method was used to determine the concentration of Tofacitinib in each plasma sample. The pharmacokinetic statistical software DAS 2.0 was used for calculation and biostatistical analysis was performed. The test results were shown in Table 11 and Figure 2.

**Table 11 Main pharmacokinetic parameters of Tofacitinib single administration**

| | Tofacitinib coated tablet | Tofacitinib coated tablet | Tofacitinib coated tablet |
|---|---|---|---|
| | (T1) | (T2) | (T3) |
| Dosage form | coated tablet | coated tablet | coated tablet |
| Administration Route | oral | oral | oral |
| Dosage | 11 mg, Once a day | 11 mg, Once a day | 11 mg, Once a day |
| Cₘₐₓ (ng/mL) | 50±23 | 58±22 | 70±32 |
| AUC₀₋₃₆ₕ (ng·h/ml) | 404±140 | 406±123 | 324±111 |
| Tₘₐₓ (h) | 2.9[2.0~3.7] | 2.4[1.2~3.8] | 0.7[0.3~1.0] |
| Ta (h) | 8.6 | 9.7 | 5.8 |

Ta refers to the time that the blood concentration can be maintained above 17 ng/mL (IC₅₀).

From the blood concentration curves, it can be seen that the time for the testing formulations T1, T2 and T3 to reach the blood concentration of 17 ng/ml were about 50 min, 25 min and 12 min respectively.

It can be seen from Table 11 that the blood concentrations of the formulations of the present disclosure showed smooth fluctuation, and higher area under the curve of the blood concentration. The Ta values of the testing formulations T1, T2 and T3 were 8.6, 9.7 and 5.8 h, respectively. It can be seen that the formulations of the present disclosure can achieve high Ta value, showing better therapeutic effects. In particular, testing formulations T1, T2 can achieve significantly higher Ta values and higher area under the curve of blood concentration, showing a particular advantage.

## Claims

1. A solid formulation, wherein the solid formulation comprises an immediate release portion and a sustained release portion, and comprises Tofacitinib or a pharmaceutically acceptable salt thereof as active ingredients, wherein
the sustained release portion comprises a sustained release matrix material, wherein
the sustained release matrix material is about 40%-80% of the weight of the sustained release portion;
the immediate release portion comprises a film-forming material; wherein
the film-forming material is about 40%-80% of the weight of the immediate release portion;
the weight ratio of the active ingredient in the sustained release portion to the active ingredient in the immediate release portion is about 7:3-20:1.

2. The solid formulation according to claim 1, wherein
the weight ratio of the active ingredient in the sustained release portion to the active ingredient in the immediate release portion is about 7:3-19:1, preferably about 4:1-19:1, more preferably about 4:1-12:1, for example about 4:1-11:1, about 4:1-9:1 or about 4:1-10:1.

3. The solid formulation according to claim 1 or 2, wherein
the active ingredient is Tofacitinib citrate.

4. The solid formulation according to any one of claims 1-3, wherein
the sustained release matrix material is about 40%-50% or about 50%-70% of the weight of the sustained release portion.

5. The solid formulation according to any one of claims 1-4, wherein
the sustained release portion comprises a film-forming material;
preferably
the film-forming material is about 30%-70%, more preferably about 40%-60% of the weight of the sustained release portion; and/or
the film-forming material is about 50%-70% of the weight of the immediate release portion.

6. The solid formulation according to any one of claims 1-5, wherein
the active ingredient in the solid formulation has a total dissolution within 15 min of about 5-50%, preferably about 10-30%, specifically preferably about 20%;
the active ingredient in the solid formulation has a total dissolution within 1 h of about 20-60%, preferably about 30-50%;
the active ingredient in the solid formulation has a total dissolution within 3 h of about 55-95%, preferably about 60-80%; and/or
the active ingredient in the solid formulation has a total dissolution within 5 h of at least about 80%, preferably at least about 85%.

7. The solid formulation according to any one of claims 1-6, wherein
after a single administration, the time require by the solid formulation for achieving a blood concentration of 17 ng/ml is about 10-60 min, preferably about 25-45 min, specifically preferably about 20-30 min; and/or
after a single administration, the duration time of the solid formulation for achieving a blood concentration over 17 ng/ml is about 8-12 h, preferably about 9-11 h.

8. The solid formulation according to any one of claims 1-7, which is in the form of a tablet, a granule, a pellet or a capsule;
preferably the tablet is a coated tablet or an osmotic pump tablet, the coated tablet or the osmotic pump tablet optionally comprises a sustained coating.

9. The solid formulation according to any one of claims 1-8, wherein
the immediate release portion and the sustained release portion each independently comprise one or more pharmaceutically excipients selected from the group consisting of a sustained release matrix material, a film-forming material, a binder, a filler, a disintegrant, a lubricant;
preferably, the sustained release matrix material is one or more selected from the group consisting of: hydroxypropyl methyl cellulose, hydroxypropyl cellulose, hydroxyethyl cellulose, polyoxyethylene, glyceryl behenate, polymethylmethacrylate, acrylic resin, povidone, xanthan gum, sodium alginate, calcium alginate, carbomer, chitin, polyethylene, polysiloxane, preferably hydroxyethyl cellulose or glyceryl behenate;
preferably, the film-forming material is one or more selected from the group consisting of: cellulose acetate, ethyl cellulose, hydroxypropyl methyl cellulose, acrylic resin, preferably ethyl cellulose or cellulose acetate;
preferably, the binder is one or more selected from the group consisting of: polyethylene glycol, starch, hydroxypropyl cellulose, hydroxypropyl methyl cellulose, pregelatinized starch,polyvinylpyrrolidone, gum arabic, gelatin, preferably hydroxypropyl cellulose;
preferably, the filler is one or more selected from the group consisting of: starch, pregelatinized starch, modified starch, microcrystalline cellulose, silicified microcrystalline cellulose, low-substituted hydroxypropyl cellulose glucose, sucrose, lactose, sorbitol, mannitol, erythritol, calcium carbonate and calcium hydrophosphate, preferably mannitol, microcrystalline cellulose, silicified microcrystalline cellulose, preferably microcrystalline cellulose, lactose or mannitol;
preferably, the disintegrant is one or more selected from the group consisting of: microcrystalline cellulose, carboxymethyl cellulose, copovidone, croscarmellose sodium, calcium carboxymethylcellulose, sodium carboxymethyl starch, low-substituted hydroxypropyl cellulose, hydroxymethyl starch, alginic acid, sodium alginate, guar gum, corn starch and magnesium aluminum silicate, preferably copovidone;
preferably, the lubricant is one or more selected from the group consisting of: magnesium stearate, stearic acid, stearate, sodium stearyl fumarate, sodium lauryl sulfate, polyethylene glycol, sodium benzoate, sucrose fatty acid ester, micronized silica gel, talcum powder, glyceryl monostearate, glyceryl behenate, glyceryl palmitostearate, stearic acid, hydrogenated vegetable oil, preferably magnesium stearate or glyceryl behenate.

10. The solid formulation according to any one of claims 1-9, wherein
the solid formulation is a bilayer tablet, and comprises the active ingredient, the filler, the disintegrant, the lubricant and the sustained release matrix material; or
the solid formulation is a coated tablet, and comprises the active ingredient, the filler, the sustained release matrix material, the lubricant, and the film-forming material; or
the solid formulation is an osmotic pump tablet, and comprises the active ingredient, the filler, the sustained release matrix material, the lubricant, and the film-forming material; or
the solid formulation is a capsule, and comprises the active ingredient, the filler, the binder, the lubricant, and the film-forming material.

11. The solid formulation according to any one of claims 1-10, wherein
the sustained release portion comprises the film-forming material, and
the active ingredient of the immediate release portion is within the film-forming material.

12. A pharmaceutical composition, comprising the solid formulation according to any one of claims 1-11.

13. The solid formulation according to any one of claims 1-11 or the pharmaceutical composition according to claim 12, for use in the treatment or prevention of a disease comprising lupus, multiple sclerosis, rheumatoid arthritis, psoriatic arthritis, type I diabetes and diabetic complications, cancer, asthma, atopic dermatitis, autoimmune thyroid disease, moderate-to-severe active ulcerative colitis, Crohns' disease, ankylosing spondylitis, juvenile idiopathic arthritis, immune system disorder baldness, vitiligo, systemic lupus erythematosus, Alzheimer's disease and leukemia.

14. A process for preparing the solid formulation according to any one of claims 1-11, comprising
1) combining the active ingredient with one or more pharmaceutically acceptable excipients to give the immediate release portion,
2) combining the active ingredient with one or more pharmaceutically acceptable excipients to give the sustained release portion,
3) combing the immediate release portion and the sustained release portion to give the solid formulation;
wherein
step 1) and step 2) are conducted in any order independently;
the active ingredients and the excipients in step 1) and step 2) are used independently.

## Patentansprüche

1. Feste Formulierung, wobei die feste Formulierung einen Anteil für sofortige Freisetzung und einen Anteil für verzögerte Freisetzung sowie Tofacitinib oder ein pharmazeutisch akzeptables Salz davon als aktive Bestandteile umfasst, wobei
der Anteil für verzögerte Freisetzung ein Matrixmaterial für verzögerte Freisetzung umfasst, wobei
das Matrixmaterial für verzögerte Freisetzung etwa 40-80 Gew.-% des Anteils für verzögerte Freisetzung ausmacht;
der Anteil für sofortige Freisetzung ein filmbildendes Material umfasst; wobei
das filmbildende Material etwa 40-80 Gew.-% des Anteils für sofortige Freisetzung ausmacht;
das Gewichtsverhältnis des aktiven Bestandteils im Anteil für verzögerte Freisetzung zum aktiven Bestandteil im Anteil für sofortige Freisetzung etwa 7:3-20:1 beträgt.

2. Feste Formulierung gemäß Anspruch 1, wobei
das Gewichtsverhältnis des aktiven Bestandteils im Anteil für verzögerte Freisetzung zum aktiven Bestandteil im Anteil für sofortige Freisetzung etwa 7:3-19:1, bevorzugt etwa 4:1-19:1, mehr bevorzugt etwa 4:1-12:1, beispielsweise etwa 4:1-11:1, etwa 4:1-9:1 oder etwa 4:1-10:1 beträgt.

3. Feste Formulierung gemäß Anspruch 1 oder 2, wobei
der aktive Bestandteil Tofacitinib-Citrat ist.

4. Feste Formulierung gemäß einem der Ansprüche 1-3, wobei
das Matrixmaterial für die verzögerte Freisetzung etwa 40-50 Gew.-% oder etwa 50-70 Gew.-% des Anteils für verzögerte Freisetzung ausmacht.

5. Feste Formulierung gemäß einem der Ansprüche 1-4, wobei
der Anteil für verzögerte Freisetzung ein filmbildendes Material umfasst,
wobei bevorzugt
das filmbildende Material etwa 30-70 Gew.-%, mehr bevorzugt etwa 40-60 Gew.-% des Anteils für verzögerte Freisetzung ausmacht; und/oder
das filmbildende Material etwa 50-70 Gew.-% des Anteils für sofortige Freisetzung ausmacht.

6. Feste Formulierung gemäß einem der Ansprüche 1-5, wobei
der aktive Bestandteil in der festen Formulierung innerhalb von 15 min eine Gesamtlöslichkeit von etwa 5-50 %, bevorzugt etwa 10-30 %, besonders bevorzugt etwa 20 % aufweist;
der aktive Bestandteil in der festen Formulierung innerhalb von 1 h eine Gesamtlöslichkeit von etwa 20-60 %, bevorzugt etwa 30-50 %, aufweist;
der aktive Bestandteil in der festen Formulierung innerhalb von 3 h eine Gesamtlöslichkeit von etwa 55-95 %, bevorzugt etwa 60-80% aufweist; und/oder
der aktive Bestandteil in der festen Formulierung innerhalb von 5 h eine Gesamtlöslichkeit von mindestens etwa 80 %, bevorzugt mindestens etwa 85%, aufweist.

7. Feste Formulierung gemäß einem der Ansprüche 1-6, wobei
nach einer einmaligen Verabreichung die Zeit, die die feste Formulierung benötigt, um eine Blutkonzentration von 17 ng/ml zu erreichen, etwa 10-60 min, bevorzugt etwa 25-45 min, besonders bevorzugt etwa 20-30 min beträgt; und/oder
nach einer einmaligen Verabreichung die Zeitdauer, die die feste Formulierung benötigt, um eine Blutkonzentration von über 17 ng/ml zu erreichen, etwa 8-12 h, bevorzugt etwa 9-11 h beträgt.

8. Feste Formulierung gemäß einem der Ansprüche 1-7 in Form einer Tablette, eines Granulats, eines Pellets oder einer Kapsel;
wobei die Tablette bevorzugt eine beschichtete Tablette oder eine osmotische Pumpentablette ist, wobei die beschichtete Tablette oder die osmotische Pumpentablette gegebenenfalls eine Retardbeschichtung umfasst.

9. Feste Formulierung gemäß einem der Ansprüche 1-8, wobei
der Anteil für sofortige Freisetzung und der Anteil für verzögerte Freisetzung jeweils unabhängig voneinander einen oder mehrere pharmazeutische Hilfsstoffe umfassen, die aus der Gruppe ausgewählt sind, bestehend aus einem Matrixmaterial für verzögerte Freisetzung, einem filmbildenden Material, einem Bindemittel, einem Füllstoff, einem Sprengmittel und einem Gleitmittel;
das Matrixmaterial für die verzögerte Freisetzung bevorzugt aus einem oder mehreren Materialien aus der Gruppe ausgewählt ist, bestehend aus: Hydroxypropylmethylcellulose, Hydroxypropylcellulose, Hydroxyethylcellulose, Polyoxyethylen, Glycerylbehenat, Polymethylmethacrylat, Acrylharz, Povidon, Xanthangummi, Natriumalginat, Calciumalginat, Carbomer, Chitin, Polyethylen, Polysiloxan, bevorzugt Hydroxyethylcellulose oder Glycerylbehenat;
das filmbildende Material bevorzugt aus einem oder mehreren Materialien aus der Gruppe ausgewählt ist, bestehend aus: Celluloseacetat, Ethylcellulose, Hydroxypropylmethylcellulose, Acrylharz, bevorzugt Ethylcellulose oder Celluloseacetat;
das Bindemittel bevorzugt aus einem oder mehreren Materialien aus der Gruppe ausgewählt ist, bestehend aus: Polyethylenglykol, Stärke, Hydroxypropylcellulose, Hydroxypropylmethylcellulose, vorgelatinierter Stärke, Polyvinylpyrrolidon, Gummi arabicum, Gelatine, bevorzugt Hydroxypropylcellulose;
der Füllstoff bevorzugt aus einem oder mehreren Materialien aus der Gruppe ausgewählt ist, bestehend aus: Stärke, vorgelatinierter Stärke, modifizierter Stärke, mikrokristalliner Cellulose, silikatierter mikrokristalliner Cellulose, niedrigsubstituierter Hydroxypropylcellulose, Glukose, Saccharose, Laktose, Sorbit, Mannit, Erythrit, Calciumcarbonat und Calciumhydrogenphosphat, bevorzugt Mannit, mikrokristalline Cellulose, silikatierte mikrokristalline Cellulose, bevorzugt mikrokristalline Cellulose, Laktose oder Mannit;
das Sprengmittel bevorzugt aus einem oder mehreren Materialien aus der Gruppe ausgewählt ist, bestehend aus: mikrokristalliner Cellulose, Carboxymethylcellulose, Copovidon, Croscarmellose-Natrium, Calciumcarboxymethylcellulose, Natriumcarboxymethylstärke, niedrigsubstituierter Hydroxypropylcellulose, Hydroxymethylstärke, Alginsäure, Natriumalginat, Guarkernmehl, Maisstärke und Magnesiumaluminiumsilikat, bevorzugt Copovidon;
das Gleitmittel bevorzugt aus einem oder mehreren Materialien aus der Gruppe ausgewählt ist, bestehend aus: Magnesiumstearat, Stearinsäure, Stearat, Natriumstearylfumarat, Natriumlaurylsulfat, Polyethylenglykol, Natriumbenzoat, Saccharosefettsäureestern, mikronisiertem Silikagel, Talkumpuder, Glycerylmonostearat, Glycerylbehenat, Glycerylpalmitostearat, Stearinsäure, hydriertem Pflanzenöl, bevorzugt Magnesiumstearat oder Glycerylbehenat.

10. Feste Formulierung gemäß einem der Ansprüche 1-9, wobei
die feste Formulierung eine zweischichtige Tablette ist und den aktiven Bestandteil, den Füllstoff, das Sprengmittel, das Gleitmittel und das Material für die Matrix zur verzögerten Freisetzung umfasst; oder
die feste Formulierung eine beschichtete Tablette ist und den aktiven Bestandteil, den Füllstoff, das Material für die Matrix zur verzögerten Freisetzung, das Gleitmittel und das filmbildende Material umfasst; oder
die feste Formulierung eine osmotische Pumpentablette ist und den aktiven Bestandteil, den Füllstoff, das Material für die Matrix zur verzögerten Freisetzung, das Gleitmittel und das filmbildende Material umfasst; oder
die feste Formulierung eine Kapsel ist und den aktiven Bestandteil, den Füllstoff, das Bindemittel, das Gleitmittel und das filmbildende Material umfasst.

11. Feste Formulierung gemäß einem der Ansprüche 1-10, wobei
der Teil für verzögerte Freisetzung das filmbildende Material umfasst und
der aktive Bestandteil des Teils für sofortige Freisetzung sich innerhalb des filmbildenden Materials befindet.

12. Pharmazeutische Zusammensetzung, umfassend die feste Formulierung gemäß einem der Ansprüche 1-11

13. Feste Formulierung gemäß einem der Ansprüche 1-11 oder pharmazeutische Zusammensetzung gemäß Anspruch 12 zur Verwendung bei der Behandlung oder Vorbeugung einer Erkrankung, umfassend Lupus, Multiple Sklerose, rheumatoide Arthritis, psoriatische Arthritis, Typ-I-Diabetes und diabetische Komplikationen, Krebs, Asthma, atopische Dermatitis, Autoimmunerkrankungen der Schilddrüse, mittelschwere bis schwere aktive Colitis ulcerosa, Morbus Crohn, ankylosierende Spondylitis, juvenile idiopathische Arthritis, durch Störungen des Immunsystems bedingten Haarausfall, Vitiligo, systemischen Lupus erythematodes, Alzheimer-Krankheit und Leukämie.

14. Verfahren zur Herstellung der festen Formulierung gemäß einem der Ansprüche 1-11, umfassend
1) Kombinieren des aktiven Bestandteils mit einem oder mehreren pharmazeutisch akzeptablen Hilfsstoffen, um den Anteil für sofortige Freisetzung zu erhalten,
2) Kombinieren des aktiven Bestandteils mit einem oder mehreren pharmazeutisch akzeptablen Hilfsstoffen, um den Anteil für verzögerte Freisetzung zu erhalten,
3) Kombinieren des Anteils für sofortige Freisetzung und des Anteils für verzögerte Freisetzung, um die feste Formulierung zu erhalten,
wobei
Schritt 1) und Schritt 2) in beliebiger Reihenfolge unabhängig voneinander durchgeführt werden;
die aktiven Bestandteile und die Hilfsstoffe in Schritt 1) und Schritt 2) unabhängig voneinander verwendet werden.

## Revendications

1. Formulation solide, la formulation solide comprenant une portion à libération immédiate et une portion à libération prolongée, et comprenant du tofacitinib ou un sel pharmaceutiquement acceptable de celui-ci en tant que principes actifs, et dans laquelle
la portion à libération prolongée comprend un matériau de matrice à libération prolongée,
ledit matériau de matrice à libération prolongée représentant environ 40 % à 80 % du poids de la portion à libération prolongée ;
la portion à libération immédiate comprend un matériau filmogène,
ledit matériau filmogène représentant environ 40 % à 80 % du poids de la portion à libération immédiate ;
le rapport pondéral entre le principe actif présent dans la portion à libération prolongée et le principe actif présent dans la portion à libération immédiate est d'environ 7:3 à 20:1.

2. Formulation solide selon la revendication 1, dans laquelle
le rapport pondéral entre le principe actif présent dans la portion à libération prolongée et le principe actif présent dans la portion à libération immédiate est d'environ 7:3 à 19:1, de préférence d'environ 4:1 à 19:1, plus préférablement d'environ 4:1 à 12:1, par exemple d'environ 4:1 à 11:1, d'environ 4:1 à 9:1 ou d'environ 4:1 à 10:1.

3. Formulation solide selon la revendication 1 ou 2, dans laquelle
le principe actif est le citrate de tofacitinib.

4. Formulation solide selon l'une quelconque des revendications 1 à 3, dans laquelle
le matériau de matrice à libération prolongée représente environ 40 % à 50 % ou environ 50 % à 70 % du poids de la portion à libération prolongée.

5. Formulation solide selon l'une quelconque des revendications 1 à 4, dans laquelle
la portion à libération prolongée comprend un matériau filmogène,
et dans laquelle de préférence
le matériau filmogène représente environ 30 % à 70 %, de préférence environ 40 % à 60 %, du poids de la portion à libération prolongée, et/ou
le matériau filmogène représente environ 50 % à 70 % du poids de la portion à libération immédiate.

6. Formulation solide selon l'une quelconque des revendications 1 à 5, dans laquelle
le principe actif de la formulation solide présente une dissolution totale en 15 minutes d'environ 5 à 50 %, de préférence d'environ 10 à 30 %, plus préférablement d'environ 20 %,
le principe actif de la formulation solide présente une dissolution totale en 1 heure d'environ 20 à 60 %, de préférence d'environ 30 à 50 %,
le principe actif de la formulation solide présente une dissolution totale en 3 heures d'environ 55 à 95 %, de préférence d'environ 60 à 80 %, et/ou
le principe actif de la formulation solide présente une dissolution totale en 5 heures d'au moins environ 80 %, de préférence d'au moins environ 85 %.

7. Formulation solide selon l'une quelconque des revendications 1 à 6, dans laquelle
après une seule administration, le temps nécessaire à la formulation solide pour atteindre une concentration sanguine de 17 ng/ml est d'environ 10 à 60 minutes, de préférence d'environ 25 à 45 minutes, plus préférablement d'environ 20 à 30 minutes, et/ou
après une seule administration, la durée nécessaire à la formulation solide pour atteindre une concentration sanguine supérieure à 17 ng/ml est d'environ 8 à 12 h, de préférence d'environ 9 à 11 h.

8. Formulation solide selon l'une quelconque des revendications 1 à 7, se présentant sous la forme d'un comprimé, d'une granule, d'un granulé ou d'une gélule,
le comprimé étant de préférence un comprimé enrobé ou un comprimé à pompe osmotique, le comprimé enrobé ou à pompe osmotique comprenant éventuellement un enrobage à libération prolongée.

9. Formulation solide selon l'une quelconque des revendications 1 à 8, dans laquelle
la portion à libération immédiate et la portion à libération prolongée comprennent chacune indépendamment un ou plusieurs excipients pharmaceutiquement acceptables sélectionnés dans le groupe constitué par un matériau de matrice à libération prolongée, un matériau filmogène, un liant, un agent de charge, un délitant et un lubrifiant,
le matériau de matrice à libération prolongée consistant de préférence en un ou plusieurs matériaux choisis dans le groupe constitué par : hydroxypropylméthylcellulose, hydroxypropylcellulose, hydroxyéthylcellulose, polyoxyéthylène, béhénate de glycéryle, polyméthacrylate de méthyle, résine acrylique, povidone, gomme xanthane, alginate de sodium, alginate de calcium, carbomère, chitine, polyéthylène, polysiloxane ; de préférence l'hydroxyéthylcellulose ou le béhénate de glycéryle,
le matériau filmogène consistant de préférence en un ou plusieurs matériaux choisis dans le groupe constitué par : acétate de cellulose, éthylcellulose, hydroxypropylméthylcellulose, résine acrylique ; de préférence l'éthylcellulose ou l'acétate de cellulose,
le liant consistant de préférence en un ou plusieurs matériaux choisis dans le groupe constitué par : polyéthylène glycol, amidon, hydroxypropylcellulose, hydroxypropylméthylcellulose, amidon prégélatinisé, polyvinylpyrrolidone, gomme arabique, gélatine ; de préférence l'hydroxypropylcellulose,
l'agent de charge consistant de préférence en un ou plusieurs matériaux choisis dans le groupe constitué par : amidon, amidon prégélatinisé, amidon modifié, cellulose microcristalline, cellulose microcristalline silicifiée, hydroxypropylcellulose faiblement substituée, glucose, saccharose, lactose, sorbitol, mannitol, érythritol, carbonate de calcium et hydrophosphate de calcium ; de préférence le mannitol, la cellulose microcristalline, la cellulose microcristalline silicifiée ; de préférence la cellulose microcristalline, le lactose ou le mannitol,
le délitant consiste de préférence en un ou plusieurs matériaux choisis dans le groupe constitué par : cellulose microcristalline, carboxyméthylcellulose, copovidone, croscarmellose sodique, carboxyméthylcellulose de calcium, amidon carboxyméthylé sodique, hydroxypropylcellulose faiblement substituée, amidon hydroxyméthylé, acide alginique, alginate de sodium, gomme de guar, amidon de maïs et silicate de magnésium et d'aluminium ; de préférence la copovidone,
le lubrifiant consistant de préférence en un ou plusieurs matériaux choisis dans le groupe constitué par : stéarate de magnésium, acide stéarique, stéarate, fumarate de stéaryle sodique, sulfate de lauryle sodique, polyéthylène glycol, benzoate de sodium, ester d'acide gras de saccharose, gel de silice micronisé, poudre de talc, monostéarate de glycéryle, béhénate de glycéryle, palmitostéarate de glycéryle, acide stéarique, huile végétale hydrogénée ; de préférence le stéarate de magnésium ou le béhénate de glycéryle.

10. Formulation solide selon l'une quelconque des revendications 1 à 9, dans laquelle
la formulation solide est un comprimé bicouche et comprend le principe actif, l'agent de charge, le délitant, le lubrifiant et le matériau de matrice à libération prolongée, ou
la formulation solide est un comprimé enrobé et comprend le principe actif, l'agent de charge, le matériau de matrice à libération prolongée, le lubrifiant et le matériau filmogène, ou
la formulation solide est un comprimé à pompe osmotique et comprend le principe actif, l'agent de charge, le matériau de matrice à libération prolongée, le lubrifiant et le matériau filmogène, ou
la formulation solide est une gélule et comprend le principe actif, l'agent de charge, le liant, le lubrifiant et le matériau filmogène.

11. Formulation solide selon l'une quelconque des revendications 1 à 10, dans laquelle
la portion à libération prolongée comprend le matériau filmogène, et
le principe actif de la portion à libération immédiate se trouve à l'intérieur du matériau filmogène.

12. Composition pharmaceutique, comprenant la formulation solide selon l'une quelconque des revendications 1 à 11.

13. Formulation solide selon l'une quelconque des revendications 1 à 11 ou composition pharmaceutique selon la revendication 12, destinée à être utilisée dans le traitement ou la prévention d'une maladie comprenant le lupus, la sclérose en plaques, la polyarthrite rhumatoïde, l'arthrite psoriasique, le diabète de type I et ses complications, le cancer, l'asthme, la dermatite atopique, les maladies thyroïdiennes auto-immunes, la colite ulcéreuse active modérée à sévère, la maladie de Crohn, la spondylarthrite ankylosante, l'arthrite juvénile idiopathique, la calvitie d'origine immunitaire, le vitiligo, le lupus érythémateux systémique, la maladie d'Alzheimer et la leucémie.

14. Procédé de préparation de la formulation solide selon l'une quelconque des revendications 1 à 11, comprenant
1) le mélange du principe actif avec un ou plusieurs excipients pharmaceutiquement acceptables afin d'obtenir la portion à libération immédiate,
2) le mélange du principe actif avec un ou plusieurs excipients pharmaceutiquement acceptables afin d'obtenir la portion à libération prolongée,
3) le mélange de la portion à libération immédiate et de la portion à libération prolongée afin d'obtenir la formulation solide,
et dans lequel
les étapes 1) et 2) sont réalisées dans n'importe quel ordre et de manière indépendante,
les principes actifs et les excipients des étapes 1) et 2) sont utilisés indépendamment les uns des autres.
